# EUROPEAN PATENT APPLICATION

(11) **EP 4 729 062 A1**
(43) Date of publication of application: **22.04.2026**
(21) Application number: 24823751.3
(22) Date of filing: 14.06.2024
(51) Int. Cl.: A61K 31/713, A61K 31/502, A61K 31/5025, A61K 47/64, A61K 45/06, A61P 35/00, C12N 15/113

(54) **PHARMACEUTICAL COMPOSITION FOR PREVENTING OR TREATING TARGETED CANCER COMPRISING BRCA-SPECIFIC SIRNA AS ACTIVE INGREDIENT**

(30) Priority: 16.06.2023 US 202363508864 P
(71) Applicant: Korea Institute of Science and Technology, Seoul 02792 (KR); Dana Farber Cancer Institute, Inc., Boston, Massachusetts 02215 (US)
(72) Inventor: RYU, Ju Hee, Seoul 02792 (KR); KWON, Ick Chan, Seoul 02792 (KR); KO, Youngji, Yongin-si Gyeonggi-do 16842 (KR); ROBERTS, Thomas M, Cambridge, Massachusetts 02138 (US); ZHAO, Jean J, Brookline, Massachusetts 02446 (US); DING, Liya, Buffalo, New York 14221 (US)
(74) Representative: advotec.
(86) International application number: PCT/KR2024/008247
(87) International publication number: WO 2024/258235

(57) **Abstract**

Provided is BRCA-specific siRNA for enhancing the sensitivity of cancer cells to PARP inhibitors. According to the present disclosure, the siRNA can induce cancer cell death together with the PARP inhibitors in patients with wild-type BRCA genes with a low therapeutic effect of the PARP inhibitors, and the fusion protein-siRNA complex for siRNA delivery is up-taken into cells via CD47, and thus more specifically delivered to cancer cells while up-taken with high efficiency, thereby maximizing a desired cell death effect.

## Description

### TECHNICAL FIELD

The present disclosure provides a wild-type BRCA-specific siRNA for improving the sensitivity of cancer cells to a PARP inhibitor, and specifically, provides a pharmaceutical composition for preventing or treating cancer for co-administration with a PARP inhibitor including the siRNA as an active ingredient, and the like.

### BACKGROUND ART

BRCA1 and BRCA2 genes play an important role in a DNA repair process, and mutations occurring in the genes are known to be associated with an increased risk of breast cancer and ovarian cancer. In the case of ovarian cancer, about 10% of all ovarian cancer patients correspond to hereditary ovarian cancer, and in the case of breast cancer, about 7% of patients are known to be caused by a genetic predisposition, and most hereditary breast cancer and ovarian cancer are caused by mutations of BRCA 1 and BRCA2 genes.

Cancer cells with BRCA1/2 mutation are known to repair DNA by a base excision repair (BER) mechanism, and a PARP protein is essentially required for activation of a BER pathway. Accordingly, Olaparib, Veliparib, Niraparib, and Rucaparib have been approved as PARP inhibitors for the treatment of breast and ovarian cancers with BRCA mutations. In addition, recently, it has been shown that the RARP inhibitor induces a strong anti-tumor immune response of STING-mediated induction in BRCA-deficient ovarian cancer cells. However, the BRCA1/2 mutation is identified in only about 10% of all ovarian and breast cancer patients, and in about 90% of ovarian cancer and breast cancer patients with an unmutated wild-type BRCA gene, an anticancer effect of the PARP inhibitor is lower, and better therapy for these cancers is still needed.

Meanwhile, small interfering RNA (siRNA) is a small RNA fragment of 18 to 27 nt in size produced by cleavage of double-stranded RNA by a Dicer, and may be used by binding specifically to mRNA having a complementary sequence to inhibit the expression of the mRNA. Various RNAi-based drugs have been approved by the FDA for the knockdown of target genes that cause diseases, but siRNA-based drugs for cancer treatment need to solve the problem of effective delivery into cells and target cell-specific delivery. In other words, it is important for siRNA-based anticancer agents to have a delivery system that can effectively deliver the siRNA preferentially into a target cell together with the siRNA sequence that induces effective knockdown of the target gene.

CD47 is a cell membrane protein that interacts with SIRPα to protect cells from macrophages. The cancer cells overexpress CD47 to evade the innate immune system, and furthermore, especially in ovarian cancer, the overexpression of CD47 is highly correlated with poor prognosis. Recently, the present inventors confirmed that siRNA may be specifically delivered to cancer cells using SIRPα.

The present inventors made intensive research to develop an effective therapeutic approach for ovarian cancer and breast cancer with a wild-type BRCA gene and then completed the present disclosure.

### [Prior Art Documents]

### [Patent Document]

KR 10-2228271

### [Non-Patent Document]

Engineered SIRP Variants as Immunotherapeutic Adjuvants to Anticancer Antibodies. Science, 341(6141), 88-91.

### DISCLOSURE OF THE INVENTION

### Technical Goals

The present disclosure has been made in an effort to enhance a cancer cell death effect of a PARP inhibitor for cancers having wild-type BRCA1 and/or BRCA2 genes in carcinomas in which a high correlation between a BRCA gene mutation and the development of cancer has been identified.

Accordingly, an object of the present disclosure is to provide a pharmaceutical composition for co-administration with a PARP inhibitor as a pharmaceutical composition for treating cancer including the siRNA as an active ingredient.

Another object of the present disclosure is to provide a pharmaceutical composition for enhancing the effect of a PARP inhibitor including the siRNA as an active ingredient.

Yet another object of the present disclosure is to provide a fusion protein-siRNA complex in which siRNA is conjugated to a fusion protein in which a SIRPα protein and a linker peptide cleaved by a lysosomal enzyme are covalently linked to each other in order to specifically deliver the siRNA to cancer cells with high efficiency.

However, technical objects of the present disclosure are not limited to the aforementioned purpose and other objects which are not mentioned may be clearly understood by those skilled in the art from the following description.

### Technical Solutions

According to an aspect, there is provided a pharmaceutical composition for preventing or treating cancer including breast cancer susceptibility gene (BRCA) 1-specific small interfering RNA (siRNA) and/or BRCA2-specific siRNA as an active ingredient.

As an embodiment of the present disclosure, the pharmaceutical composition may be used for co-administration with a poly ADP-ribose polymerase (PPAR) inhibitor.

As another embodiment of the present disclosure, the BRCA1-specific siRNA may be an oligonucleotide of 15 to 31 nt in length capable of complementarily binding to BRCA1 mRNA, and may specifically include or consist of a nucleotide sequence represented by SEQ ID NO: 3.

As another embodiment of the present disclosure, the BRCA2-specific siRNA may be an oligonucleotide of 15 to 31 nt in length capable of complementarily binding to BRCA2 mRNA, and may specifically include or consist of a nucleotide sequence represented by SEQ ID NO: 5.

According to another aspect, there is provided a composition for enhancing the sensitivity of cancer cells to a PPAR inhibitor, including the BRCA1-specific siRNA and/or BRCA2-specific siRNA as an active ingredient.

As an embodiment of the present disclosure, the PARP inhibitor may be at least one selected from the group consisting of Olaparib, Veliparib, Niraparib, Rucaparib, and Talazoparib.

As another embodiment of the present disclosure, the cancer may be a cancer known to have a high correlation between a BRCA gene mutation and development, metastasis, or recurrence of the cancer, and non-limiting examples thereof may include ovarian cancer, breast cancer, prostate cancer, and the like.

As another embodiment of the present disclosure, the cancer may be a carcinoma known to have a low cancer cell death effect of the PARP inhibitor in patients with an unmutated BRCA gene or wild-type BRCA gene in carcinomas that have a high correlation between the BRCA gene mutation and the development, metastasis, or recurrence of the cancer.

In another embodiment of the present disclosure, the cancer may be a cancer having an unmutated BRCA1 gene or wild-type BRCA1 gene in carcinomas that have a high correlation between the BRCA gene mutation and the development, metastasis, or recurrence of the cancer.

In another embodiment of the present disclosure, the cancer may be a cancer having an unmutated BRCA2 gene or wild-type BRCA2 gene in carcinomas that have a high correlation between the BRCA gene mutation and the development, metastasis, or recurrence of the cancer.

In another embodiment of the present disclosure, the cancer may be a cancer having unmutated BRCA1 and BRCA2 genes or wild-type BRCA1 and BRCA2 genes in carcinomas that have a high correlation between the BRCA gene mutation and the development, metastasis, or recurrence of the cancer.

Meanwhile, the siRNA of the present disclosure may be provided by conjugating the siRNA to a fusion protein in which a SIRPα protein and a linker peptide are covalently linked to each other to deliver siRNA specifically to cancer cells. The siRNA conjugated to the fusion protein is taken up into cells via the interaction between SIRPα and CD47 proteins, which may act more effectively on cancer cells overexpressing CD47.

According to yet another aspect, there is provided a fusion protein-siRNA complex for effectively delivering siRNA to cancer cells overexpressing CD47.

As an embodiment of the present disclosure, the linker peptide constituting the fusion protein is not limited as long as the linker peptide may be cleaved in a cell to release siRNA from the complex, and in a specific experiment of the present disclosure, a linker peptide having a sequence cleaved by a lysosomal enzyme was used.

As another embodiment of the present disclosure, the SIRPα protein may be modified to improve the affinity with CD47.

According to still another aspect, there is provided a method for preventing or treating cancer including (1) administering the BRCA1-specific siRNA and/or BRCA2-specific siRNA to a subject, and (2) administering a PARP inhibitor to the subject.

As an embodiment of the present disclosure, in the method, the subject may be a patient with ovarian cancer, breast cancer, or prostate cancer with an unmutated BRCA1 gene, an unmutated BRCA2 gene, or unmutated BRCA1 and BRCA2 genes.

According to still yet another aspect, there is provided a use of the BRCA1-specific siRNA and/or BRCA2-specific siRNA for preparing drugs for enhancing a cancer cell death effect of a PARP inhibitor.

### EFFECTS

According to the present disclosure, the BRCA-specific siRNA can enhance the sensitivity to a PARP inhibitor in cancers in which the therapeutic effect of a PARP inhibitor is lower than expected because the cancer cells retain about 80% or more expression of a wild-type BRCA gene. In addition, the siRNA of the present disclosure is provided to be fused with the SIRPα protein to exhibit little cytotoxicity while having excellent cancer cell targeting ability and intracellular delivery ability, and thus, the siRNA is expected to be useful with the PARP inhibitor for the treatment of cancer patients with wild-type BRCA genes.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
FIG. 1 is a schematic diagram of a mechanism of vSIRPα-siBRCA;
FIG. 2A is a result of BRCA1 siRNA #1 to #3's silencing effect, and FIG. 2B is a result of BRCA2 siRNA #1 to #3's silencing effect.
FIG. 3A illustrates confirming the insertion of a vSIRPα-linker gene into a pET28a vector, FIG. 3B illustrates confirming the expression of a vSIRPα-linker in cells transformed with the vector by Coomassie gel staining, and FIG. 3C illustrates re-confirming the expression of the vSIRPα-linker by RP-HPLC;
FIG. 4A is a schematic diagram of a preparation process and a structure of vSIRPα-siBRCA;
FIG. 4B illustrates confirming the formation of vSIRPα-siBRCA by Nucleic acid staining;
FIG. 5A illustrates a result of ID8 cells treated with vSIRPα-siBRCA-Cy5 and observed under a confocal microscope after nuclear staining, and FIG. 5B illustrates a result of ID8 cells pre-incubated with an anti-CD47 antibody, treated with vSIRPα-siBRCA-Cy5 and observed under a confocal microscope after nuclear staining;
FIG. 6A illustrates a result of confirming the cell viability of ID8 cells treated with vSIRPα-siBRCA1 or Lipo-siBRCA1. FIG. 6B illustrates a result of confirming the cell viability of ID8 cells treated with vSIRPα-siBRCA1, vSIRPα-siBRCA2, or vSIRPα-siBRCA1/2;
FIG. 7A illustrates confirming the expression level of a BRCA1 gene in ID8 cells treated with vSIRPα-siBRCA1 for each concentration by RT-PCR analysis. FIG. 7B illustrates confirming the expression level of a BRCA2 gene in ID8 cells treated with vSIRPα-siBRCA2 for each concentration by RT-PCR analysis. FIGS. 7C and 7D illustrate confirming the expression level of the BRCA1 or BRCA2 gene in ID8 cells through RT-PCR analysis after treatment with 400 nM vSIRPα-siBRCA1 and vSIRPα-siBRCA2, respectively;
FIG. 8 illustrates a result of flow cytometry for γH2AX by treating ID8 cells with vSIRPα-siBRCA1, vSIRPα-siBRCA2, or vSIRPα-siBRCA1/2 and then adding a PARP inhibitor; and
FIG. 9A to FIG. 9D illustrates a result of treating ID8 cells incubated with vSIRPα-siBRCA with a PARP inhibitor (Olaparib or Talazoparib) and performing IC₅₀ analysis through MTS assay.

### BEST MODE FOR CARRYING OUT THE INVENTION

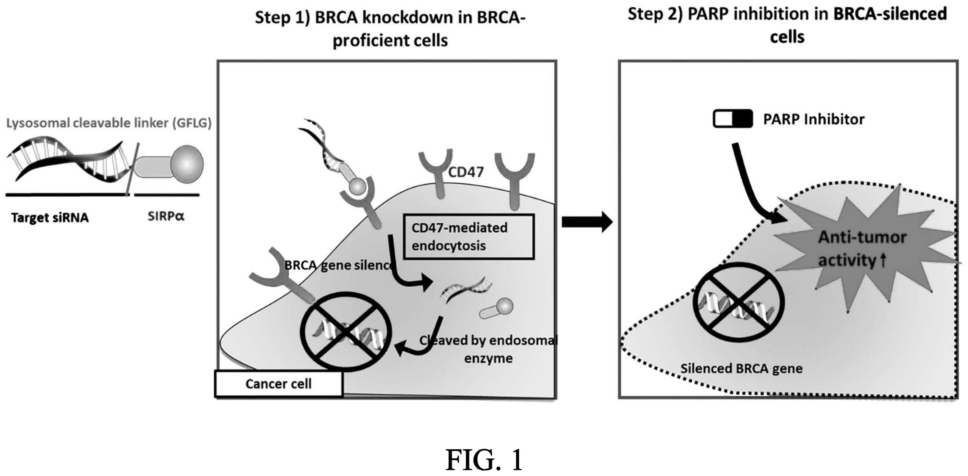

The present inventors intended to improve the sensitivity of cancer cells to a PARP inhibitor by using siRNA targeting BRCA mRNA for effective treatment of ovarian cancer and breast cancer having wild-type BRCA genes, and developed a SIRPα-siBRCA conjugate to sufficiently deliver the siRNA specifically to cancer cells (FIG. 1).

BRCA1 and BRCA2 genes play an important role in a DNA repair process, and in the case of cancer cells with BRCA1/2 mutations, DNA is repaired through a BER mechanism that essentially requires a PARP protein. Therefore, in the case of hereditary breast cancer and ovarian cancer caused by mutations in the BRCA1 and BRCA2 genes, a strong antitumor effect may be obtained by the PARP inhibitor, but in breast cancer and ovarian cancer with unmutated wild-type BRCA genes, there is a problem that the anticancer effect of the PARP inhibitor is low.

The present inventors confirmed that the expression level of yH2AX, a marker of DNA damage, was increased when cells knocked down or knocked out with BRCA1 and/or BRCA2 genes were treated with the PARP inhibitor. Furthermore, it was confirmed that when the expression of BRCA2 or BRCA1 was inhibited in BRCA1 or BRCA2 deleted cells, respectively, the level of cancer cell death by the PARP inhibitor was increased.

Accordingly, the present inventors provide siRNA capable of inhibiting the expression of the BRCA1 or BRCA2 gene in order to enhance the anticancer effect by the PARP inhibitor in cancer having the wild-type BRCA1 and/or BRCA2 genes.

In the present disclosure, 'short interfering RNA (siRNA)' means double-stranded RNA capable of inducing RNAi that inhibits the activity of the gene. In the present disclosure, siRNA refers to siRNA capable of inhibiting the expression of the wild-type BRCA1 or wild-type BRCA2 gene, and in the present disclosure, the siRNA that inhibits the expression of the wild-type BRCA1 gene is used interchangeably with the term "siBRCA1", and the siRNA that inhibits the expression of the wild-type BRCA2 gene is used interchangeably with the term "siBRCA2".

Unless otherwise specified in this specification, the BRCA gene means an unmutated wild-type BRCA gene.

The present inventors searched for siRNAs that more effectively inhibit the expression of wild-type BRCA1 gene or wild-type BRCA2 gene, and selected the siRNAs in Table 3 below.

The BRCA-specific siRNA of the present disclosure may be chemically or enzymatically synthesized. The preparation method of siRNA is not particularly limited, and methods known in the art may be used. For example, the preparation method includes a method of directly chemically synthesizing siRNA, a synthesis method of siRNA using *in vitro* transcription, a method of cleaving long double-stranded RNA synthesized by *in vitro* transcription using an enzyme, an expression method through intracellular delivery of an shRNA expression plasmid or viral vector, an expression method through intracellular delivery of a polymerase chain reaction (PCR)-induced siRNA expression cassette, etc., but is not limited thereto.

Meanwhile, the introduction of the above-described siRNA into the cells may be performed using a microinjection method, a calcium phosphate co-precipitation method, an electroporation method, a liposome method, etc. However, the present inventors have developed a "fusion protein-siRNA complex" in which the siRNA is conjugated to a fusion protein in which an SIRPα protein and a linker peptide are covalently linked to each other to improve the cancer cell targeting ability and intracellular delivery ability of the siRNA.

Accordingly, in the present disclosure, the BRCA1 or BRCA2-specific siRNA may be provided in the form of the fusion protein-siRNA complex described above.

The fusion protein-siRNA complex of the present disclosure may be quickly delivered into cells with high efficiency compared to direct administration of siRNA.

In the fusion protein of the present disclosure, the linker peptide may be a peptide having a sequence cleaved by a lysosomal enzyme preferably to be cleavable so that the siRNA may be released from the complex in the cytoplasm, and more preferably to be cleaved when introduced into the cells.

The fusion protein-siRNA complex of the present disclosure is easily soluble in a physiological solution to be easily absorbed and have excellent bioavailability. Specifically, the complex of the present disclosure facilitates cancer cell-specific intracellular delivery and has excellent potential stability compared to the case of using the siRNA or SIRPα protein alone. In addition, since there is no risk of uncontrolled replication and there is no need to use viral or non-viral vectors for siRNA delivery, the complex may be very stable because there is no need to worry about interference due to transfection and potential side effects. In addition, compared to using a delivery system such as a vector, synthesis is very cheap and delivery efficiency is very good (maximum preventive or therapeutic effect is achieved by administering low concentrations of siRNA and SIRPα protein), which has a great advantage in terms of economy.

The siRNA used in the preparation of the fusion protein-siRNA complex of the present disclosure is not limited, as long as the siRNA may silence the wild-type BRCA gene (BRCA1 or BRCA2 gene) or inhibit the expression of the gene. However, in terms of the silencing effect, it is preferable to use siRNA including or consisting of a nucleotide sequence represented by SEQ ID NO: 3 or 5 or an experimentally determined sequence that is more effective. Maleimide may be bound to the 3'-terminal of the siRNA for conjugation with the fusion protein.

In the present disclosure, the "complex" includes the following fusion protein and siRNA, and refers to a covalent complex formed by chemical bonds between the fusion protein and the siRNA. The chemical bonds are not particularly limited as long as the chemical bonds are generally used in the art, but preferably, in order to bind between cysteine residues of the fusion protein and the siRNA, a derivative including a maleimide group may be bound to the terminal of the siRNA via a linker, which is meant that thiol of the cysteine residue present in the fusion protein reacts with the maleimide group of the siRNA to form a bond.

The siRNA of the present disclosure inhibits the expression of BRCA1 and/or BRCA2 in cancer cells to improve the sensitivity of the cancer cells to the PARP inhibitor and enhance an anticancer effect of the PARP inhibitor. Thus, the composition including the siRNA of the present disclosure is provided for co-administration with the PARP inhibitor, and the composition including the siRNA may be administered simultaneously, sequentially, or alternately with the PARP inhibitor, but preferably, the PARP inhibitor is administered about 24 hours after administration of the siRNA.

That is, the pharmaceutical composition for preventing or treating cancer of the present disclosure increases the sensitivity of the cancer cells to the PARP inhibitor, and thus, in the present disclosure, the pharmaceutical composition for preventing or treating cancer is preferably understood as a pharmaceutical adjuvant composition for preventing or treating cancer.

The composition of the present disclosure may be formulated by including at least one pharmaceutically acceptable carrier in addition to the above-described active ingredients for administration.

In the composition formulated with a liquid solution, the pharmaceutically acceptable carrier is suitable for sterilization and living bodies and may use saline, sterilized water, Ringer's solution, buffered saline, albumin injection solution, dextrose solution, maltodextrin solution, glycerol, ethanol, and a mixture of at least one of these ingredients, and if necessary, may add other general additives such as antioxidants, buffers, bacteriostatic agents, and the like.

In addition, the pharmaceutical composition for treating or preventing cancer may be prepared using pharmaceutically suitable and physiologically acceptable adjuvants in addition to the active ingredients. As the adjuvants, solubilizers such as excipients, disintegrants, sweeteners, binders, coating agents, expanding agents, lubricants, slip modifiers, or flavoring agents may be used.

In the present disclosure, the formulation of the pharmaceutical composition is not particularly limited, but preferably provided with injection formulations, such as an aqueous solution, a suspension, or an emulsion by additionally adding a diluent, a dispersing agent, a surfactant, a binder, and a lubricant.

Since the pharmaceutical composition for treating or preventing cancer of the present disclosure targets receptors of the cancer cells and already has specificity for cancer cells, a route of administration is not particularly limited. However, preferably, the pharmaceutical composition may be administered by a general method through intravenous, intraarterial, intraperitoneal, intramuscular, intraarterial, intraperitoneal, intrasternal, transdermal, nasal, intranasal, inhalational, topical, rectal, oral, intraocular, or intradermal route. Most preferably, the pharmaceutical composition may be injected through intravenous injection, but is not particularly limited thereto. Depending on a purpose, the pharmaceutical composition may be administered through intraperitoneal, intramuscular, subcutaneous, oral, intranasal, intrapulmonary, or intrarectal route.

The composition of the present disclosure may further include suitable carriers, excipients, and diluents which are commonly used in the preparation of pharmaceutical composition and the like. The 'carrier' is a compound that facilitates the addition of the compound into a cell or tissue. The 'diluent' is a compound diluted in water that not only stabilizes a biologically active form of a target compound, but also dissolves the compound.

The carriers, excipients, and diluents that may be included in the pharmaceutical composition of the present disclosure may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. When the pharmaceutical composition is formulated, the formulation may be prepared by using diluents or excipients, such as a filler, an extender, a binder, a wetting agent, a disintegrating agent, and a surfactant, which are generally used.

The term "administration" used herein means providing a predetermined composition of the present disclosure to a subject by any suitable method.

The dosage of the composition of the present disclosure may vary depending on the sex, body weight, age, etc. of the subject, and will depend above all on the condition of a subject to be treated, a specific category or type of a disease to be treated, a route of administration, and properties of a therapeutic agent to be used.

In the present disclosure, the "subject" is not limited as long as the subject is mammals, but is preferably a cancer patient with an unmutated BRCA1 and/or BRCA2 gene, and a cancer patient with a wild-type BRCA1 and/or wild-type BRCA2 gene. The cancer patient having the wild-type BRCA gene is a patient showing a lower effect than an expected cancer treatment effect by the PARP inhibitor in a BRCA null tumor, and may be a patient having about 80% or more of the wild-type BRCA gene expression.

Hereinafter, embodiments will be described in detail with reference to the accompanying drawings. However, since various modifications may be made to the embodiments, the scope of the present disclosure is not limited or restricted by these embodiments. It should be understood that all modifications, equivalents, and substitutes for the embodiments are included in the scope of the present disclosure.

The terms used in the example embodiments are used for the purpose of description only, and should not be construed to be limited. The singular expression includes the plural expression unless the context clearly dictates otherwise. In the present specification, it should be understood that the term "comprising" or "having" indicates that a feature, a number, a step, an operation, a component, a part, or the combination thereof described in the specification is present, but does not exclude a possibility of presence or addition of one or more other features, numbers, steps, operations, components, parts, or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present application.

In addition, in the description with reference to the accompanying drawings, like components designate like reference numerals regardless of reference numerals and a duplicated description thereof will be omitted. In describing the example embodiments, a detailed description of related known technologies will be omitted if it is determined that they unnecessarily make the gist of the example embodiments unclear.

### [Experimental methods and materials]

### 1. Materials

A DNA plasmid encoding enhanced vSIRPα was purchased from Cosmo Genetech (Seoul, Republic of Korea). BRCA1 siRNA, BRCA2 siRNA, and Cy5-conjugated BRCA1 siRNA were purchased from Integrated DNA Technologies (IDT; Coralville, IA, USA). Kanamycin, isopropyl β-D-1-thiogalactopyranoside (IPTG), maleimide-PEG2-NHS ester, and cathepsin B were purchased from Sigma-Aldrich (St. Louis, MO, USA). A pET28a vector and Lipofectame 3000 were purchased from Invitrogen (Carlsbad, CA, USA), and T4 DNA ligase and Nde1 and HindIII restriction enzymes were purchased from New England Biolabs (Ipswich, MA, USA). LB broth, phenol chloroform isoamylalchol, and bromophenol blue were purchased from Sigma-Aldrich. An RNeasy Mini kit and Ni-NTA agarose were purchased from Qiagen (Hilden, Germany). For some cell experiments, an RPMI medium, fetal bovine serum (FBS), and a penicillin-streptomycin mixture (Pen-Strep) were purchased from Gibco (Carlsbad, CA, USA). An anti-mouse CD47 antibody (MIAP301) and a PE anti-H2A.X Phospho (Ser139) were provided by BioLegend (San Diego, CA, USA), and an iScriptTM Reverse Transcription Super mix was provided by Bio-Rad Laboratories (Hercules, CA, USA). An SYBR Green PCR Master Mix was purchased from Life Technologies (Carlsbad, CA, USA). The CellTiter 96 Aqueous One Solution Cell Proliferation Assay was provided by (Madison, WI, USA). Olaparib was purchased from Santa Cruz Biotechnology (Dallas, TX, USA), and Talazoparib (BMN673) was purchased from Selleck Chemical (Houston, TX, USA).

### 2. Preparation of vSIRPα-linker

To prepare a fusion protein using a vSIRPα protein, a lysosomal cleavable linker (GFLG) peptide, and an siRNA junction region, the following sequence was inserted into a C-terminal gene of vSIRPα to prepare a vSIRPα-linker DNA fragment: CATATGGAAGAGGAGCTGCAGATCATCCAGCCTGACAAGTCCGTGCTGGTCGCT GCTGGTGAAACTGCCACTCTGCGTTGTACGATTACCAGCCTGTTCCCGGTGGGTC CAATCCAGTGGTTCCGTGGTGCTGGTCCGGGTCGTGTTCTGATCTACAACCAGCG TCAAGGTCCGTTCCCGCGTGTAACTACCGTTAGCGATACCACGAAGCGTAACAAC ATGGACTTTTCCATCCGCATTGGCAATATTACCCCGGCCGACGCGGGCACCTACT ATTGCATCAAATTTCGCAAAGGCTCCCCGGATGATGTAGAATTTAAATCTGGCGC AGGCACCGAACTGTCTGTTCGCGCAAAACCGGTCGACGGTGGGTTTCTGGGTGG GGGAGGATGCGGTTGAAAGCTT (SEQ ID NO: 1). The underlined region was a GFLG coding sequence.

To obtain recombinant vSIRPα from *E. coli* BL21, a pET28a-vSIRPα-linker vector was prepared by inserting a vSIRPα-linker DNA fragment into the pET28a vector, and then transformed into *E. coli* BL21. Colonies were selected and incubated in LB medium, and plasmid DNA was isolated by a standard method, and then the plasmid DNA was electrophoresed on a 0.8% agarose gel to confirm a DNA composition of the vSIRPα-linker plasmid. Protein expression was induced by incubating the transformed *E. coli* BL21 at 20°C for 14 to 16 hours under a 0.5 mM IPTG condition. The next day, the cell culture solution was centrifuged at 5,000 rpm for 15 minutes to obtain *E. coli* BL21, and the cells were sonicated for 2 hours to be lysed. A cell lysed sample was centrifuged at 13,000 rpm for 10 min at 4°C and purified using a Ni-NTA column. After purification, the levels of vSIRPα isolated were confirmed by performing SDS-PAGE and HPLC.

### 3. Preparation of siBRCA

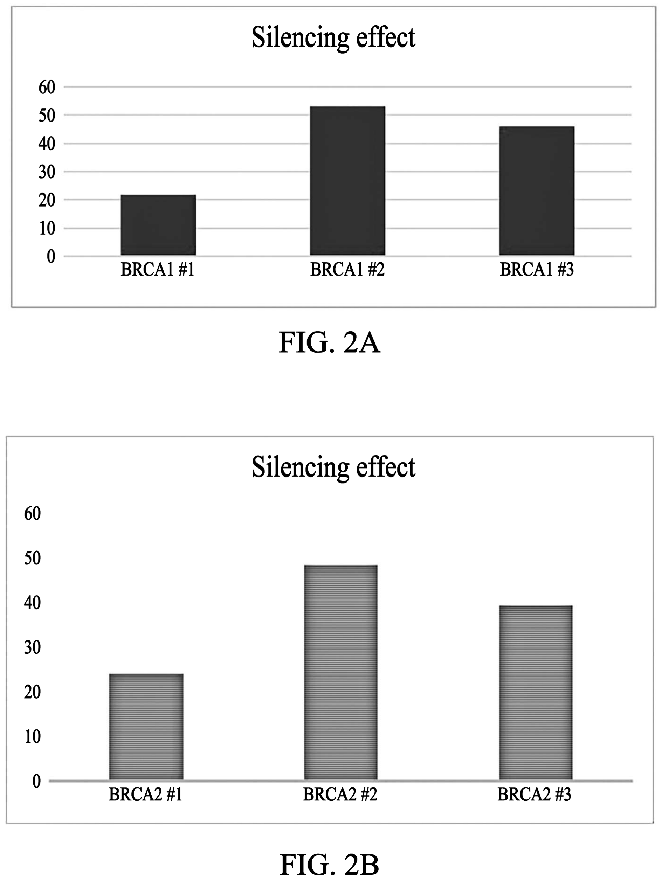

First, we designed and manufactured three types of siRNA for inhibiting the expression of BRCA1 or BRCA2. Table 1 below is shown specific sequence information of three types of BRCA1 siRNA, and Table 2 below is shown specific sequence information of three types of BRCA2 siRNA. The results of measuring the silencing effect of BRCA1 siRNA and BRCA2 siRNA using the TriFECTa kit (IDT) are shown in FIG. 2. Based on the silencing effect result, we selected optimized BRCA1 siRNA and BRCA2 siRNA, which is shown in table 3 below. In Tables 1 to 3 below, * indicated deoxyribonucleotide (DNA).

**[Table 1]**

| | | Sequence | SEQ ID NO: |
|---|---|---|---|
| BRCA1 siRNA #1 | Sense | CUUGAACCAAUCAGUAGAAAUCC*A*A | 2 |
| | Antisense | UUGGAUUUCUACUGAUUGGUUCAAGUG | 3 |
| BRCA1 siRNA #2 | Sense | GAGCUUCAAAUCGAUAGUUGUGG*T*A | 6 |
| | Antisense | UACCACAACUAUCGAUUUGAAGCUCAG | 7 |
| BRCA1 siRNA #3 | Sense | GAUCUGUGGUUUGACUUAAAAUC*A*G | 8 |
| | Antisense | CUGAUUUUAAGUCAAACCACAGAUCUC | 9 |

**[Table 2]**

| | | Sequence | SEQ ID NO: |
|---|---|---|---|
| BRCA2 siRNA #1 | Sense | AUGCUAUUGAGAAUAUCGAUACA*T*T | 4 |
| | Antisense | AAUGUAUCGAUAUUCUCAAUAGCAUGC | 5 |
| BRCA2 siRNA #2 | Sense | GUCAAACCUUUCUGGUCUAAAUG*A*A | 10 |
| | Antisense | UUCAUUUAGACCAGAAAGGUUUGACUG | 11 |
| BRCA2 siRNA #3 | Sense | GCAUGAUUGGUUCUGCUAGAGCU*C*C | 12 |
| | Antisense | GGAGCUCUAGCAGAACCAAUCAUGCAG | 13 |

**[Table 3]**

| | | Sequence | SEQ ID NO: |
|---|---|---|---|
| BRCA1 siRNA | Sense | 5'-Amine- CUUGAACCAAUCAGUAGAAAU CC*A*A-3' (*; DNA) | 2 |
| | Antisense | 5’- UUGGAUUCUACUGUGGUUCAA GUG-3’ | 3 |
| BRCA2 siRNA | Sense | 5’-Amine- AUGCUAUUGAGAAUAUCAUCA *T*T-3’ | 4 |
| | Antisense | 5’- AAUGUAUCGAUUCAUUCAAUA GCAUGC-3’ | 5 |

### 4. Synthesis and characteristics of vSIRPα-siBRCA

Amine-modified siBRCA (10 nmol) was conjugated with maleimide-NHS (300 nmol) in 50 mM HEPES (pH 7.4) at 4°C for 4 hours. The maleimide-conjugated siBRCA was purified using a NAP-10 column and lyophilized. Subsequently, recombinant vSIRPα (10 nmol) including a linker was conjugated with the maleimide-conjugated siBRCA (10 nmol) through a thiol-maleimide coupling reaction for 4 hours under 50 mM HEPES (pH 7.0 to 7.4) conditions. Coupling was confirmed by performing a 12% SDS-PAGE of the product, after which the gel was stained with GelRed Nucleic Acid dye (10,000X) and scanned with GelDotTMXR+ (Bio-Rad) to confirm vSIRPα-siBRCA synthesis.

### 5. Intracellular uptake and cytotoxicity

A mouse ovarian cancer cell line, termed ID8, was incubated in RPMI 1640 containing 10% FBS, 1% Pen-Strep, 1% L-glutamine, 0.5% sodium pyruvate, and 1.7 µl of β-mercaptoethanol in an incubator at 37°C and 5% CO₂. 2 × 10⁵ cells/dish of cells were dispensed on a 35 mm cover glass dish.

For an intracellular uptake identification experiment, as an siBRCA incorporated into vSIRPα-siBRCA, with the siBRCA1 conjugated with Cy5 was used. To determine whether the intracellular uptake of vSIRPα-siBRCA was CD47-dependent, ID8 cells were pre-incubated with an anti-CD47 antibody for 30 minutes to block uptake via CD47. After culturing, the cells were immobilized with 4% formaldehyde, and the immobilized cells were incubated with DAPI for 10 minutes at room temperature, and the nuclei were stained and observed under a confocal microscope.

To confirm the cytotoxicity of vSIRPα-siBRCA, ID8 cells were dispensed in a 96-well microplate at 3 × 10³ cells per well and treated with vSIRPα-siBRCA1 or with lipofectamine containing siBRCA1 (Lipo-siBRCA1) at various concentrations, after 48 hours, the cell viability was confirmed using a cell proliferation assay kit. The absorbance of the solution was measured at 490 nm with a plate reader.

### 6. In vitro gene silencing

ID8 cells were dispensed in a 6-well plate at 2 × 10⁵ cells/dish, treated with PBS, siBRCA1, siBRCA1, and lipofectamine, or vSIRPα-siBRCA1 and incubated for 48 hours. The final concentration of each sample was set to 600 nM of siRNA. RNA was isolated using an RNeasy Mini kit according to the manufacturer's instructions, and cRNA was synthesized using an iScriptTM Reverse Transcription Super mix. Similarly, the ID8 cells were treated with PBS, siBRCA1, siBRCA2, and lipofectamine (Lipo-siBRCA2), or vSIRPα-siBRCA2 and incubated for 48 hours, and RNA was obtained and analyzed by RT-PCR in the same manner as described above.

### 7. DNA damage analysis by flow cytometry

A decrease in activation of a DNA repair mechanism and the potentially increased levela of DNA damage by a PARP inhibitor (PARPi) upon treatment of the experimental reagents were then measured.

The ID8 cells were dispensed in a 6-well plate at 2 × 10⁵ cells/dish and incubated for 48 hours with PBS, vSIRPα-siBRCA1, vSIRPα-siBRCA2, and vSIRPα-siBRCA1/2. Subsequently, the cells were trypsinized and dispensed in a 6-well plate at 2 × 10⁵ cells/dish, and treated with Olaparib or Talazoparib at a predetermined concentration for 24 to 48 hours.

Subsequently, the cells were detached with Trypsin-EDTA, blocked for 30 minutes using a PBS solution containing 1% FBS, and then a phospho-H2AX antibody was incubated for 2 hours. Thereafter, after PBS washing, flow cytometry was performed using a FACS device to measure phospho-H2AX antibody staining

### [Experiment Results]

### 1. Synthesis and in vitro characteristics of vSIRPα-siBRCA1/2 conjugate

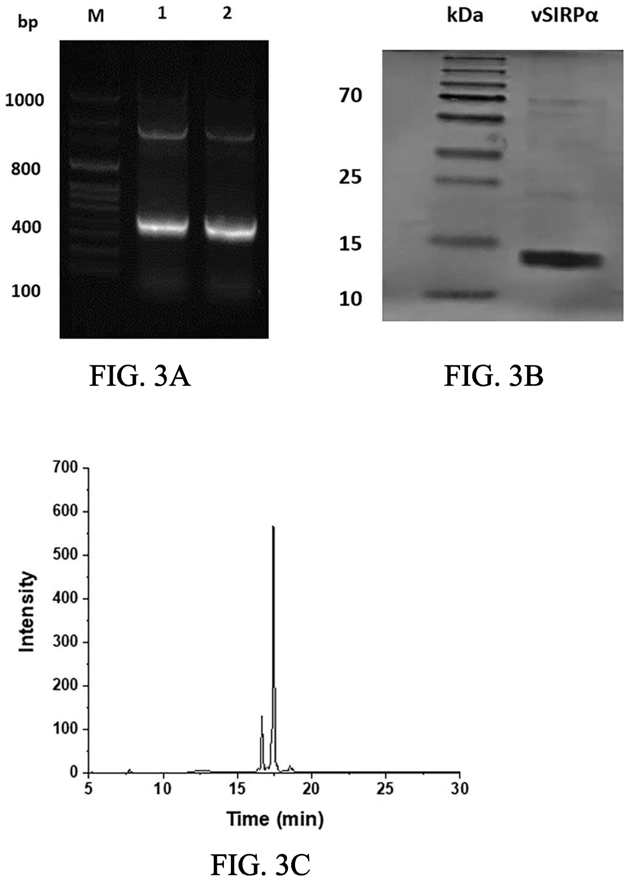

The C-terminus of vSIRPα included a linker sequence capable of binding to maleimide-modified siBRCA and being cleaved in the cytoplasm after CD47-mediated intracellular uptake. A PCR product from a plasmid prepared by cloning a DNA sequence encoding the vSIRPα-linker into a pET28a vector encoding vSIRPα was analyzed by agarose-gel electrophoresis, and as a result, it was confirmed that a 400-bp vSIRPα-linker gene was inserted into the plasmid vector as expected (FIG. 3A).

The recombinant plasmid vector was transformed into *E. coli* BL21, the transformed BL21 cells were incubated and the expression of vSIRPα was induced and confirmed by SDS-PAGE and RP-HPLC (FIGS. 3B and 3C).

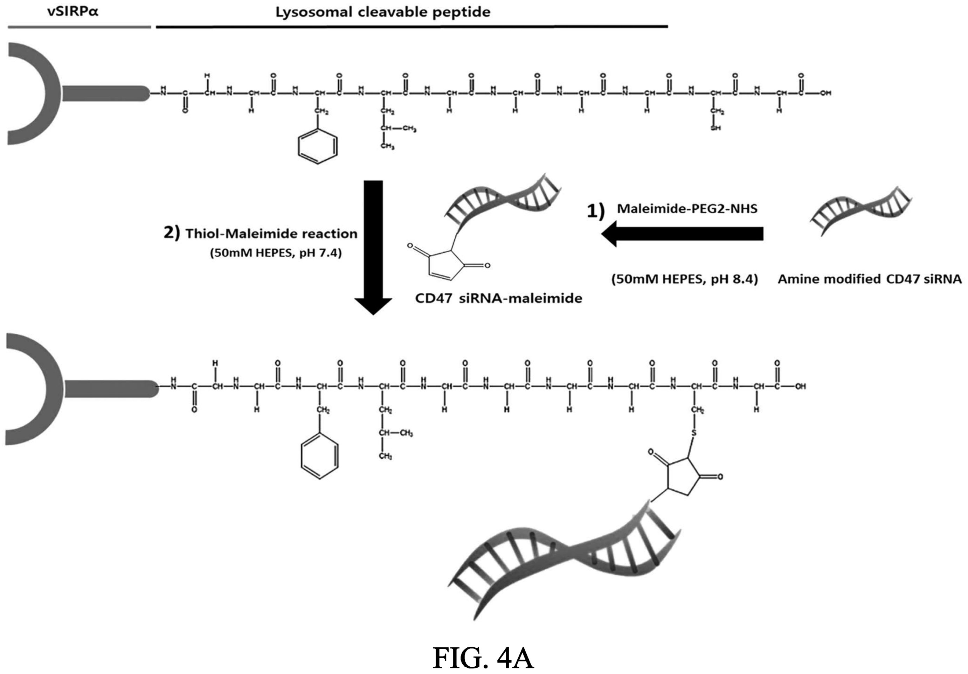

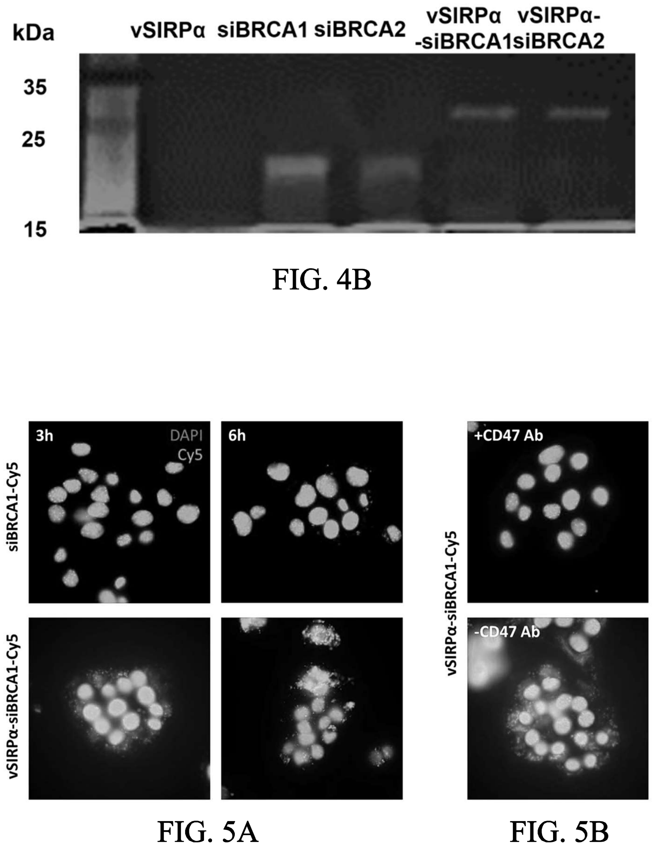

For vSIRPα-siBRCA synthesis, cysteine residues of the modified vSIRPα protein were conjugated to maleimide-conjugated siBRCA via thiol-maleimide coupling (FIG. 4A). The formation of the vSIRPα-siBRCA conjugate was confirmed by PAGE gel analysis through nuclear staining, which can be seen by a slight shift in a band due to the siBRCA conjugation compared to the vSIRPα + linker (FIG. 4B).

### 2. CD47-mediated endocytosis of vSIRPα-siBRCA conjugate in vitro

Due to a high negative charge, siRNAs generally have difficulty in penetrating through a cell membrane. For efficient siRNA delivery into cancer cells, vSIRPα-siBRCA was designed, and the absorption efficiency of the vSIRPα-siBRCA conjugate was confirmed using Cy5-conjugated siBRCA. The degree of endocytosis of the conjugate was confirmed by comparison with the Cy5-conjugated siBRCA.

ID8 cells can here represent the class of cells with a relatively high expression level of CD47. ID8 cells were treated with vSIRPα-siBRCA or siBRCA, and the cells were stained and observed under a confocal microscope (FIG. 5). Notably, the vSIRPα-siBRCA conjugate was absorbed into ID8 cancer cells with high efficiency within 3 to 6 hours, but naked siBRCA showed a very low level of absorption even after 6 hours (FIG. 5A). From the above, it can be seen that the vSIRPα-siBRCA conjugate may be up-taken into cancer cells more efficiently than siRNA and may be selectively absorbed by CD47-overexpressing cells (FIG. 5B).

### 3. Cytotoxicity and gene silencing of vSIRPα-siBRCA conjugate in vitro

The cytotoxicity of a vSIRPα-siBRCA conjugate was evaluated by confirming the cell viability after 48 hours of treatment with the vSIRPα-siBRCA conjugate to ID8 cells. The cytotoxicity of the vSIRPα-siBRCA conjugate was compared with that of cells treated with Lipo-siBRCA.

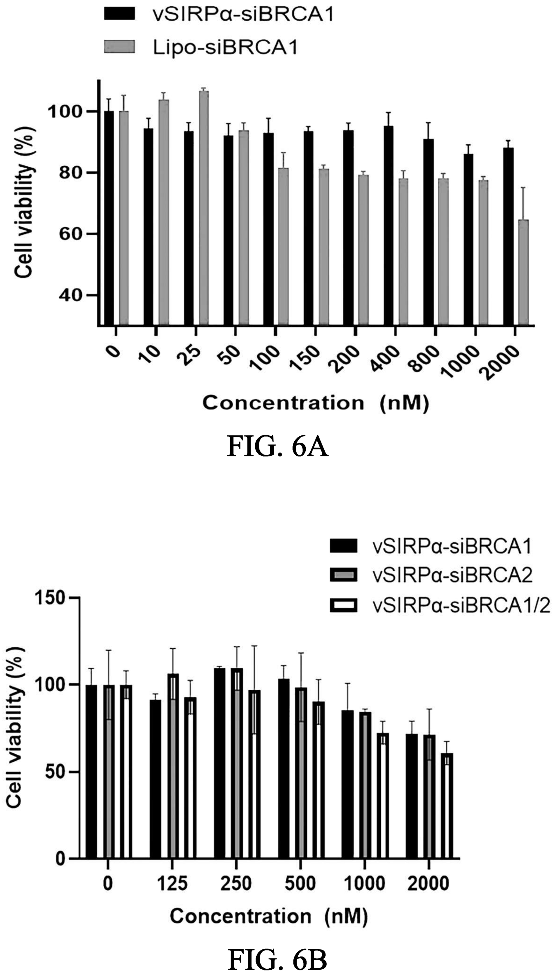

As a result, it was confirmed that the cell viability of Lipo-siBRCA1-administered cells was decreased at 100 nM and significantly lowered at 2 µM concentration, whereas vSIRPα-siBRCA1 showed no significant toxicity at a maximum of 2 µM (FIG. 6A). In addition, vSIRPα-siBRCA2 showed no significant toxicity up to 1 µM, whereas the cell viability was slightly decreased when 1 µM vSIRPα-siBRCA1 and vSIRPα-siBRCA2 were co-treated (FIG. 6B). Unlike Lipofectamine^{™}, Lipo-siBRCA showed no significant toxicity up to 2 µM.

Subsequently, ID8 cells having wild-type BRCA were treated with the vSIRPα-siBRCA conjugate to measure the expression levels of the BRCA gene. The cells were treated with the vSIRPα-siBRCA conjugate at a concentration of 200 nM or 400 nM, and for comparison, the same experiment was performed using the same amount of Lipo-siBRCA.

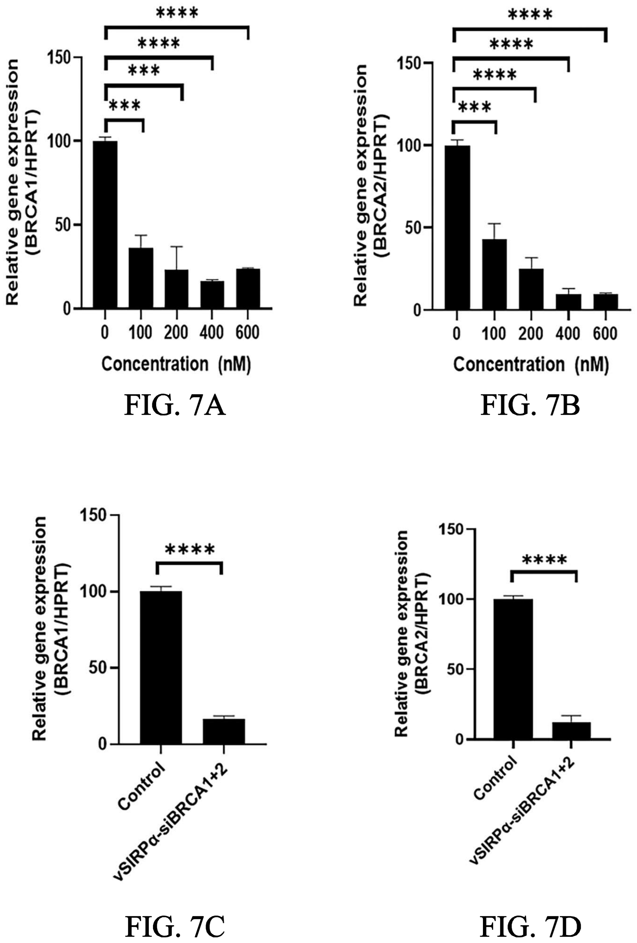

As a result, it was confirmed that 200 nM vSIRPα-siBRCA1 down-regulated the BRCA1 gene to the same extent as Lipo-siBRCA1, and that 200 nM vSIRPα-siBRCA2 also showed the same gene silencing effect (FIG. 7). Specifically, 200 nM vSIRPα-siBRCA2 reduced the expression levels of the BRCA2 gene to about 75%, 400 nM vSIRPα-siBRCA2 reduced the expression level of the BRCA2 gene to about 90%, and 400 nM vSIRPα-siBRCA1 reduced the expression level of the BRCA1 gene to about 85% (FIGS. 7A and 7B). In addition, simultaneous treatment with 400 nM vSIRPα-siBRCA1 and vSIRPα-BRCA2 was confirmed to reduce the expression levels of BRCA1 and BRCA2 genes to 85% to 90%, respectively (FIGS. 7C and 7D).

### 4. DNA damage according to treatment of PARP inhibitor in BRCA silenced cells

To evaluate the level of PARP inhibitor-mediated DNA damage in BRCA gene-silenced cells, the expression levels of phospho-H2AX (yH2AX), a DNA damage marker, were measured by flow cytometry.

ID8 cells were treated with vSIRPα-siBRCA1 and vSIRPα-siBRCA2 alone or in combination, respectively, and after 48 hours, each cell type was treated with a PARP inhibitor, Talazoparib, and incubated for 48 hours. As a result, it was confirmed that the expression levels of γH2AX were significantly increased in ID8-sgBRCA1/2 cells.

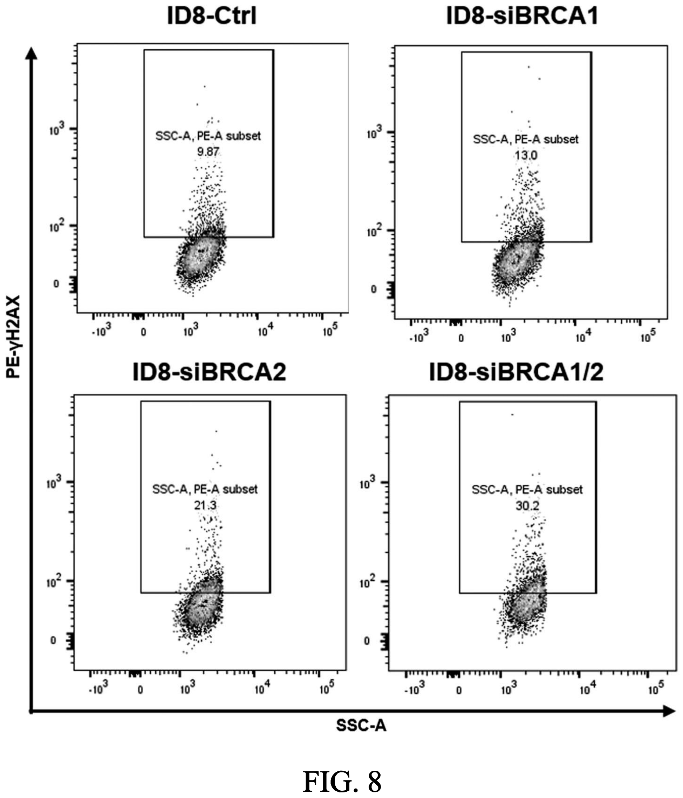

As a result of analyzing an experimental group in which BRCA silenced cells were treated with 0.01 µM Talazoparib, by flow cytometry, as compared with a control group, γH2AX was increased about 1.32-fold in BRCA1 silenced cells, 2.16-fold in BRCA2 silenced cells, and 3.06-fold in BRCA1/2 silenced cells (FIG. 8).

From the above results, it was found that silencing of the BRCA2 gene rather than BRCA1 enhanced the sensitivity of cells to the PARP inhibitor, and particularly, the sensitivity to the PARP inhibitor was most significantly improved when BRCA1/2 were silenced together.

### 5. PARP inhibitor-mediated apoptosis according to BRCA1 or BRCA2 expression silencing

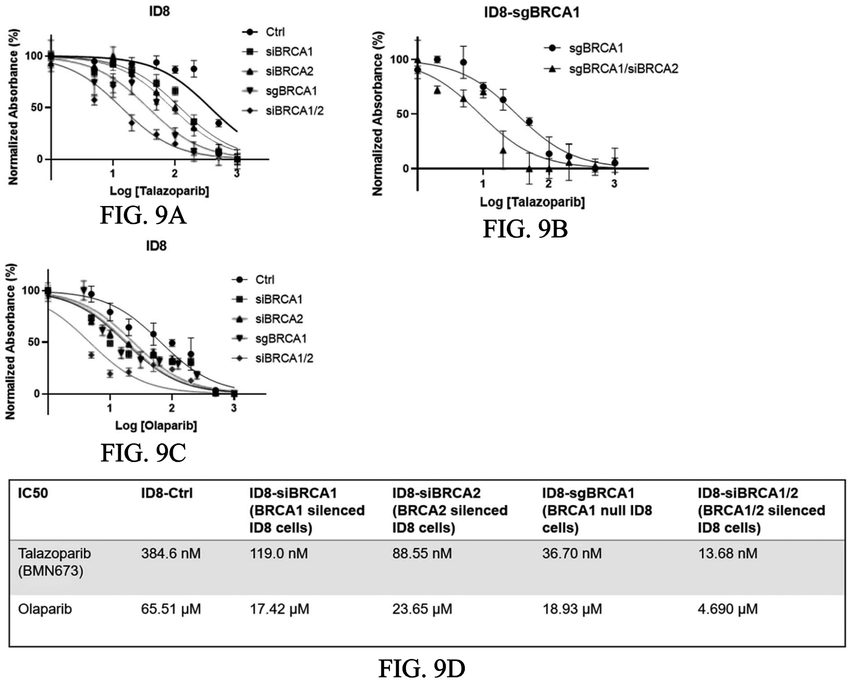

Knockdown and knockout of BRCA genes were highly correlated with the efficacy of synthetic lethal cell death induction of the PARP inhibitor. Therefore, whether the PARP inhibitor, after silencing the expression of BRCA1/2, could induce apoptosis of tumor cells was required confirmation. BRCA1 and/or BRCA2 may be effectively knocked down by treating ID8 cells with the vSIRPα-siBRCA conjugates. Therefore ID8 cells were treated with the vSIRPα-siBRCA conjugates for 48 hours and then treated with Talazoparib at various concentrations for 72 hours. Compared to BRCA normal-expressing cells, the IC₅₀ of Talazoparib was reduced 3.23-fold and 4.34-fold in BRCA1-knockdown ID8 cells and BRCA2-knockdown ID8 cells, respectively (FIGS. 9A and 9D). In particular, in the case of cells administered with vSIRPα-siBRCA1 and vSIRPα-BRCA2 together, the IC₅₀ of Talazoparib was decreased 28.11-fold. These results suggest that the effect of Talazoparib can be significantly enhanced by BRCA2 knockdown in BRCA1 null cancer cells. To confirm this, BRCA2 was knocked down in BRCA1-deficient ID8 cells, and as a result, it was confirmed that the IC₅₀ of Talazoparib was reduced about 3.6-fold by BRCA2 knockdown (FIGS. 9B and 9D).

As a result of performing the same experiment using a different PARP inhibitor, Olaparib, it was confirmed that the IC₅₀ value of Olaparib in ID8-BRCA1/2 silenced cells was about 13.97-fold lower than that of ID8 control cells, the IC₅₀ value of Olaparib in ID8-BRCA1 silenced cells was about 3.76-fold lower therethan, and the IC₅₀ value of Olaparib in BRCA2 silenced cells was about 2.77-fold lower therethan (FIGS. 9C and 9D).

From the above, it can be seen that the inhibition of the expression of BRCA1 and/or BRCA2 increases the sensitivity to the PARP inhibitors in ovarian cancer cells.

As described above, although the example embodiments have been described by the restricted drawings, the various modifications and variations can be applied on the basis of the example embodiments by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other example embodiments, and equivalents to the appended claims fall within the scope of the claims to be described below.

## Claims

1. A pharmaceutical composition for preventing or treating cancer comprising breast cancer susceptibility gene (BRCA) 1-specific small interfering RNA (siRNA) and/or BRCA2-specific siRNA as an active ingredient,
wherein the composition is administered in combination with a poly ADP-ribose polymerase (PPAR) inhibitor.

2. The pharmaceutical composition of claim 1, wherein the BRCA1-specific siRNA is an oligonucleotide of 15 to 31 nt in length capable of complementarily binding to BRCA1 mRNA, and
the BRCA2-specific siRNA is an oligonucleotide of 15 to 31 nt in length capable of complementarily binding to BRCA2 mRNA.

3. The pharmaceutical composition of claim 1, wherein the BRCA1-specific siRNA includes a nucleotide sequence represented by SEQ ID NO: 3, and
the BRCA2-specific siRNA includes a nucleotide sequence represented by SEQ ID NO: 5.

4. The pharmaceutical composition of claim 1, wherein the cancer is at least one selected from the group consisting of ovarian cancer, breast cancer, and prostate cancer.

5. The pharmaceutical composition of claim 4, wherein the cancer is a cancer including a wild-type BRCA1 gene and/or a wild-type BRCA2 gene.

6. The pharmaceutical composition of claim 1, wherein the siRNA is conjugated with a fusion protein in which a SIRPα protein and a linker peptide cleaved by a lysosomal enzyme are covalently linked to each other.

7. A composition for enhancing sensitivity of cancer cells to a PPAR inhibitor, comprising BRCA1-specific siRNA and/or BRCA2-specific siRNA as an active ingredient.

8. The composition of claim 7, wherein the BRCA1-specific siRNA includes a nucleotide sequence represented by SEQ ID NO: 3, and
the BRCA2-specific siRNA includes a nucleotide sequence represented by SEQ ID NO: 5.

9. The composition of claim 7, wherein the cancer is at least one selected from the group consisting of ovarian cancer, breast cancer, and prostate cancer.

10. The composition of claim 7, wherein the cancer cell is a cancer cell including a wild-type BRCA1 gene and/or a wild-type BRCA2 gene.

11. The composition of claim 7, wherein the siRNA is conjugated with a fusion protein in which a SIRPα protein and a linker peptide cleaved by a lysosomal enzyme are covalently linked to each other.

12. A fusion protein-siRNA complex in which siRNA is conjugated with a fusion protein in which a SIRPα protein and a linker peptide cleaved by a lysosomal enzyme are covalently linked to each other, wherein the siRNA includes a nucleotide sequence represented by SEQ ID NO: 3 or SEQ ID NO: 5.
